# EUROPÄISCHE PATENTSCHRIFT

(11) **EP 2 526 058 B1**
(45) Veröffentlichungstag und Bekanntmachung des Hinweises auf die Patenterteilung: **13.07.2016**
(21) Anmeldenummer: 11700933.2
(22) Anmeldetag: 21.01.2011
(51) Int. Cl.: C01C 3/02

(54) **EINRAUMVERDAMPFER UND DEREN VERWENDUNG BEI DER CHEMISCHEN SYNTHESE**
SINGLE-CHAMBER EVAPORATOR AND THE USE THEREOF IN CHEMICAL SYNTHESIS
ÉVAPORATEUR À CHAMBRE UNIQUE ET SON UTILISATION POUR LA SYNTHÈSE CHIMIQUE

(30) Priorität: 22.01.2010 EP 10151422
(43) Veröffentlichungstag der Anmeldung: 28.11.2012
(73) Patentinhaber: BASF SE, 67056 Ludwigshafen am Rhein (DE)
(72) Erfinder: BÖHLING, Ralf, 64653 Lorsch (DE); SCHIPPER, Michael, 67061 Ludwigshafen (DE); BIRNBACH, Stefan, 67246 Dirmstein (DE); PETERSEN, Peter, 67125 Dannstadt-Schauernheim (DE); GRITSCH, Achim, 3042 AB Rotterdam (NL); WELLISCH, Alois, 67466 Lambrecht (DE); LUYKEN, Hermann, 67069 Ludwigshafen (DE); STEINER, Albert, 67069 Ludwigshafen (DE); ZEHNER, Peter, 67273 Weisenheim am Berg (DE)
(74) Vertreter: BASF IP Association
(86) Internationale Anmeldenummer: PCT/EP2011/050805
(87) Internationale Veröffentlichungsnummer: WO 2011/089209

(56) Entgegenhaltungen:
- EP-A2- 0 209 039
- DE-A1- 10 144 891
- US-A- 2 042 451
- US-A- 2 534 000

## Beschreibung

Die Erfindung betrifft Einraumverdampfer und deren Verwendung bei der chemischen Synthese von Blausäure durch Verdampfen von Formamid und Reaktion des verdampften Formamids in einem nachgeschaltetem Reaktor.

Es ist bekannt, flüssige Edukte vor ihrer weiteren Umsetzung mit sich selbst oder anderen Edukten zu verdampfen. Die bei der Verdampfung zugeführte Energie kann insbesondere bei instabilen Verbindungen zu unerwünschten Zersetzungen und sonstigen Reaktionen führen.

So ist beispielsweise bekannt, Blausäure durch Formamid-Thermolyse zu gewinnen. Blausäure ist eine wichtige Grundchemikalie, die als Ausgangsprodukt z. B. in zahlreichen organischen Synthesen wie der Herstellung von Adipodinitril, Methacrylsäureestern, Methionin und komplexbildnern (NTA und EDTA) dient. Darüber hinaus wird es für die Herstellung von Alkalicyanide benötigt, welches im Bergbau und in der metallurgischen Industrie eingesetzt wird.

Die größte Menge an HCN wird durch Umsetzung von Methan (Erdgas) und NH3 produziert. Im Andrussow-Prozess wird simultan Luftsauerstoff hinzugegeben. Auf diese Weise läuft die Herstellung autotherm. Im Gegensatz dazu wird beim BMA-Verfahren der Degussa sauerstofffrei gearbeitet. Die endotherme katalytische Umsetzung von Methan mit NH3 wird daher extern mit einem Heizmedium (Methan oder H2) betrieben.

Ein weiteres wichtiges Verfahren zur Herstellung von HCN ist der SOHIO-Prozess. Bei dieser Ammonoxidation von Propen/Propan zu Acrylnitril entsteht ca. 10 % (bez. auf C3) HCN als Nebenausbeute.

Ein weiteres wichtiges Verfahren zur industriellen Herstellung von Blausäure ist die thermische Dehydratisierung von Formamid im Vakuum, die nach der folgenden Gleichung (1) abläuft:

HCONH₂ → HCN + H₂0 (1)

Diese Umsetzung ist von der Zersetzung des Formamids gemäß folgender Gleichung (2) unter Bildung von Ammoniak und Kohlenmonoxid begleitet:

HCONH₂ → NH₃ + CO (2)

Der gebildete Ammoniak katalysiert die Polymerisation der gewünschten Blausäure und führt somit zu einer Beeinträchtigung der Qualität der Blausäure und einer Verringerung der Ausbeute an der gewünschten Blausäure.

Die Polymerisation von Blausäure und die damit verbundene Rußbildung kann durch die Zugabe von geringen Mengen Sauerstoff in Form von Luft, wie z. B. in EP-A 0 209 039 A2 offenbart ist, unterdrückt werden. In EP-A 0 209 039 A2 ist ein Verfahren zur thermolytischen Spaltung von Formamid an hochgesinterten Aluminiumoxid- oder Aluminiumoxid-Siliziumdioxid-Formkörpern oder an hochtemperturkorrosionsfesten Chrom-Nickel- Edelstahl-Formkörpern offenbart. Der Formamidumsatz ist in diesem Verfahren nicht vollständig und des Weiteren entstehen als Nebenprodukte Ammoniak und Kohlenmonoxid gemäß Gleichung (2). Somit ist eine Abtrennung und Rückführung des Rest-Formamids notwendig, wobei schwersiedende Nebenprodukte entstehen, die aus dem Verfahren abgetrennt werden müssen. Weiterhin führt die zugesetzte Luftmenge zu einer Bildung von Kohlendioxid aus dem gemäß Gleichung (2) gebildeten Kohlenmonoxid, das mit dem gleichzeitig gebildeten Ammoniak zu festen Carbamaten reagiert und so zu schwer handhabbaren Ablagerungen und zur Korrosion in den verwendeten Anlagen führt (Feststoffproblematik). Die Spaltung wird in der Regel in Edelstahl- oder Eisenrohren durchgeführt, deren genaue Zusammensetzung nicht erwähnt wird.

US-A-2,042,451 betrifft die Dehydratisierung von Formamid zur Herstellung von HCN. Als Katalysator dient eine geheizte Oberfläche (Messing oder Eisen), die von einer dünnen katalytisch aktiven Oxidschicht aus Zn, Al, Mg, Cr. oder Sn-Oxid überzogen ist. Der Reaktionsraum ist der Zwischenraum zwischen einem zylindrischen Metallrohr und einem zylindrischen Metallstab, der in das Rohr eingeführt wurde. Wichtig ist gemäß der Beschreibung, dass kein Teil des Gases mehr als einen ½ inch von der katalytischen Oberfläche entfernt ist. Mit dem Verfahren werden Umsätze von 73 bis 89 %, bezogen auf Formamid, erzielt.

DE 1 209 561 B betrifft ein Verfahren zur Herstellung von HCN durch Spalten von Formamiddampf an Ferrioxid als Katalysator, das durch teilweise oder vollständige Bindung an Säuren unter Bildung von Salzen oder durch Kombination mit einem oder mehreren nicht flüchtigen Oxiden von 1- bis 6-wertigen Metallen desaktiviert ist. Die Katalysatoren liegen als Pellets oder als im Strangpressen gefertigte Katalysatorkörner vor.

Die Spaltung wird in einem Spaltofen mit Röhren aus einer Fe-Legierung durchgeführt, die zum Beispiel neben Fe 13 % Cr, 1 % Al, 1 % Si, < 1 % Mn und ca. 0,1 % C enthält. DE 1 000 796 B betrifft ein Verfahren zur Spaltung von Formamiddampf, wobei einem Temperaturgefälle innerhalb der Öfen zur Spaltung dadurch Rechnung getragen wird, dass die Spaltung an stückigen oder körnigen hochgebrannten eisenoxidhaltigen Silikaten oder Spinellen in einem Spaltungsraum erfolgt, dessen Wandung eine niedrigere katalytische Aktivität besitzt als die der Katalysatoren im Spaltungsraum. Die Wandung besteht zum Beispiel aus Edelstahl, der insbesondere ca. 85 % Fe und ca. 16 % Cr enthält. Der Spaltungsraum wird aus von außen beheizten Röhren gebildet.

DE 477 437 C betrifft ein Verfahren zur katalytischen Herstellung von Blausäure aus Formamid, worin Formamiddämpfe in großer Verdünnung und mit großer Geschwindigkeit in Abwesenheit von wasserabspaltenden Katalysatoren bei Temperaturen von über 300 °C über Metallkatalysatoren wie Flusseisen, V2A-Stahl, Nickel oder Aluminium geleitet werden. Gemäß einer Ausführungsform wird die Reaktion in Rohren durchgeführt, die aus katalytisch wirkendem Metall hergestellt oder mit diesem ausgekleidet sind und sonst keinen Katalysator enthalten.

In WO 2004/050587 A2 wird ein Verfahren zur Herstellung von HCN aus Formamid beschrieben, wobei die Spaltung in leeren Metall-Rohren durchgeführt wird. Dieses Verfahren hat den Vorteil, dass die Selektivität gemäß GI. 1 hoch ist, auch wenn ein geringeres Vakuum angelegt wird. Im Gegensatz zu anderen Patentschriften sind Drücke bis zu 300 mbar möglich.

US 2,534,000 A offenbart ein Verfahren zur katalytischen Umwandlung von Formamid zu Blausäure. Dazu wird eine Vorrichtung gemäß Figur 1 verwendet, die Heizelemente aufweist, die in die Wärmeisolierung eingebettet sind. Dieses Dokument unterscheidet auch nicht zwischen einer Verdampfereinheit zur Verdampfung des Formamids und einem Reaktor, in dem das verdampfte Formamid in das gewünschte Produkt Blausäure umgesetzt wird.

DE 101 44 891 A offenbart ein Verfahren zur Blausäureherstellung. Dazu wird ein Reaktor verwendet, bei dem der Wärmeeintrag durch elektrische Widerstandsheizungen der Reaktorwand des Strömungsreaktors oder von den im Strömungsreaktor angeordneten Heizkörpern erfolgt. Die thermolytische Spaltung von Formamid erfolgt dadurch, dass Formamid als Gas in reiner Form oder mit einem Inertgas verdünnt und/oder im Gemisch mit Sauerstoff eingesetzt wird.

Die Notwendigkeit, die Formamid-Thermolyse bei möglichst geringen Drücken (Vakuum) durchzuführen ist bekannt. Diese Tatsache ist ein wesentlicher Nachteil für die Wirtschaftlichkeit des FA-Verfahrens. Es hat daher nicht an Versuchen gefehlt, die Spaltung auch bei Drücken nahe Normaldruck durchzuführen. Dagegen steht aber die Instabilität des Formamids. Bei der Verdampfung ist es daher im Allgemeinen wichtig, unterhalb von Temperaturen von ca. 160 °C zu bleiben. Dies ist nur mit einem Unterdruck von ca. 150 mbar oder weniger möglich.

Gemäß US-A-2,429,262 und US-A-2,529,546 hat man daher versucht, durch Flash-Verdampfung in Kurzzeit-Verdampfern bei Normaldruck zu verdampfen und anschließend zu HCN umzusetzen. Die Selektivität war jedoch gering.

Der Einsatz von mikrostrukturierten Apparaten zur Verdampfung ist beschreiben in WO/2005/016512 A1 und WO/2006/108796 A1. Vorteile sind hohe Wärmeübertragungsleistung pro Fläche, die kompakte Bauweise und das schnelle Anfahrverhalten. Zwar gelingt mit diesem Verdampfertyp die zersetzungsfreie Verdampfung des Formamids bei Normaldruck oder leicht erhöhtem Druck, so dass sich im Verdampfer keine Polymerisate bilden, aber durch Verunreinigungen im Formamid z. B. Spuren von Metallen, oder höhersiedende Bestandteile verstopfen die Mikrokanäle, in denen eine Totalverdampfung des Formamids stattfindet. In DE 973 173 C wird daher ein Dünnschichtverdampfer mit rotierenden Wischerblättern als Verdampfer vorgeschlagen. Dieser Verdampfertyp muss einen ununterbrochenen, an der beheizten Oberfläche anliegenden Flüssigkeitsfilm, der zusammenhängend abfließt, sicherstellen. Nachteile dieses Verdampfertyps sind bezogen auf das Verdampfervolumen geringe Wärmeübertragungsflächen, da nur die beheizte Verdampferwand genutzt wird und die technisch aufwändige störungsanfällige rotierende Welle mit Rührflügeln.

Aufgabe der vorliegenden Erfindung war es daher, ein Verfahren zur Herstellung von Blausäure durch katalytische Dehydratisierung von gasförmigen Formamid in Anwesenheit von Luftsauerstoff bereitzustellen, das eine hohe Selektivität zu der gewünschten Blausäure aufweist und bei möglichst hohen Drücken (nahe Normaldruck oder höher) betrieben werden kann.

Die Erfindung betrifft ein Verfahren zur Herstellung von Blausäure durch Verdampfen von Formamid in einem Verdampfer und Überführung des verdampften Formamids in einen Reaktor C, in welchem das Formamid in der Dampfphase unter katalytischer Dehydratisierung reagiert, dadurch gekennzeichnet, dass der Verdampfer im Wesentlichen einen Behälter und im Behälter Heizrohre HR aufweist, die vorzugsweise parallel zueinander und zur Längsachse des Verdampfers verlaufen, wobei der Behälter vorzugsweise aus nur einem Raum R besteht, wobei weiter
a) die auf den Raum R bezogene äußere Fläche der Heizrohre HR wenigstens 300 m²/m³ beträgt.
b) der hydraulische Durchmesser des Raumes R 0,03 bis 1 m beträgt
c) der Raum eine Länge von 0,1 bis 5 m aufweist

Der hydraulische Durchmesser ergibt sich aus dem mit dem Faktor 4 multiplizierten Quotienten aus freier Querschnittsfläche A (Querschnittsfläche abzüglich der durch z. B, Einbauten ausgefüllten Fläche) und dem Umfang des Mantelraumes U. Der Raum R entspricht dem freien Mantelraum abzüglich der Einbauten.

### Einraumverdampfer

Die Erfindung betrifft weiterhin einen erfindungsgemäß zu verwendenden Verdampfer, im Folgenden "Einraumverdampfer" genannt, dadurch gekennzeichnet, dass der Verdampfer im Wesentlichen einen Behälter und im Behälter Heizrohre HR aufweist, die vorzugsweise parallel zueinander und zur Längsachse des Verdampfers verlaufen, wobei der Behälter vorzugsweise aus nur einem Raum R besteht, wobei weiter
a) die auf den Raum R bezogene äußere Fläche der Heizrohre HR wenigstens 300 m²/m³ beträgt.
b) der hydraulische Durchmesser des Raumes R 0,01 bis 1 m beträgt
c) der Raum eine Länge von 0,1 bis 5 m aufweist und der innere Durchmesser der Heizrohre HR 100 bis 6000 µm beträgt.

Der Mantel des Verdampfers kann grundsätzlich eine beliebige Geometrie aufweisen, ist aber vorzugsweise kreisförmig. In besonderen Ausführungsformen kann er aber auch davon abweichen und z. B. sechseckig sein. Sich während dem Verdampfungsvorgang bildende Polymerisate oder im Eduktstrom vorhandene, nicht verdampfbare Anteile, können über einen zusätzlichen Ausgang am Boden des Verdampfers abgezogen werden.

Im Einraumverdampfer wird das gesamte zu verdampfende Formamid in einem zusammenhängenden Raum verdampft. Der hydraulischen Durchmesser dieses Raumes beträgt vorzugsweise 0,03 bis 1 m, die Länge vorzugsweise 0,1 bis 5 m. Der Raum ist mit Kanälen durchzogen, die z. B. mit Dampf, dessen Druck einer Kondensationstemperatur entspricht, die mindestens 5 °C oberhalb der Siedetemperatur des Formamides liegt, betrieben werden. Der maximale Abstand eines Teilchens zur nächstliegenden Wand beträgt dabei lediglich ein Bruchteil des hydraulischen Durchmessers des Verdampferraumes, vorzugweise 100 - 1500 µm. Der Einraumverdampfer kann daher als mikro- bzw. millistrukturierter Apparat aufgefasst werden. Alternativ zu Dampf kann als Temperiermedium auch Heizgas oder Heizflüssigkeit verwendet werden, deren Eintritttemperatur in den Einraumverdampfer mindestens 5°C oberhalb der Siedetemperatur des Formamides liegt, betrieben werden. Die auf das freie Volumen des Verdampfungsraumes bezogene äußere Fläche der Kanäle, die den Verdampfungsraum durchziehen, sollte zwischen 300 bis 3000 m²/m³ betragen.

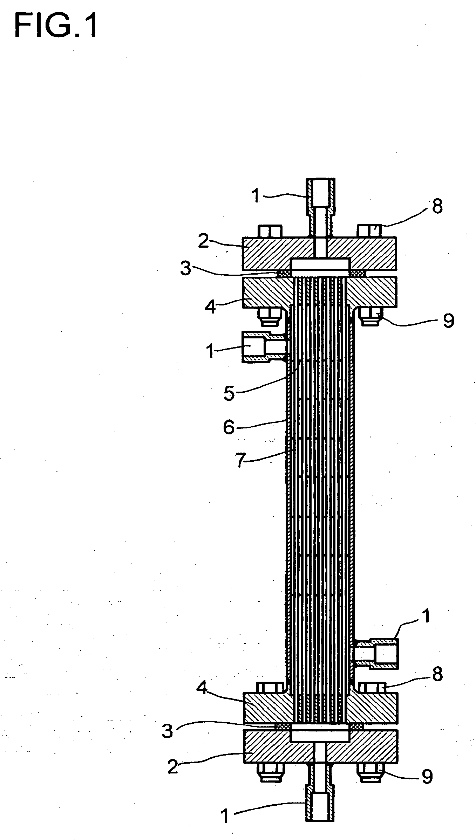

In einer besonders bevorzugten Ausführungsform wird ein Einraumverdampfer, siehe Fig. 1, verwendet, der eine Vielzahl von z. B. parallel angeordneter Temperierkanäle enthält. Eine beispielhafte Ausführungsform ist die eines Rohrbündelapparates. Im Gegensatz zu technisch üblichen Apparaten beträgt der innere Kanaldurchmesser jedoch 100 bis 6000 µm. Der Abstand zweier gegenüberliegender Kanäle sollte weniger als 3 mm betragen, um möglichst hohe verdampferraumspezifische Oberflächen von 300 bis 3000 m²/m³ zu erreichen. Diese hohen Oberflächen sind notwendig, um Formamidverweilzeiten, bezogen auf den flüssig hold up des Verdampfungsraumes, von weniger als 60 sec. zu erreichen.

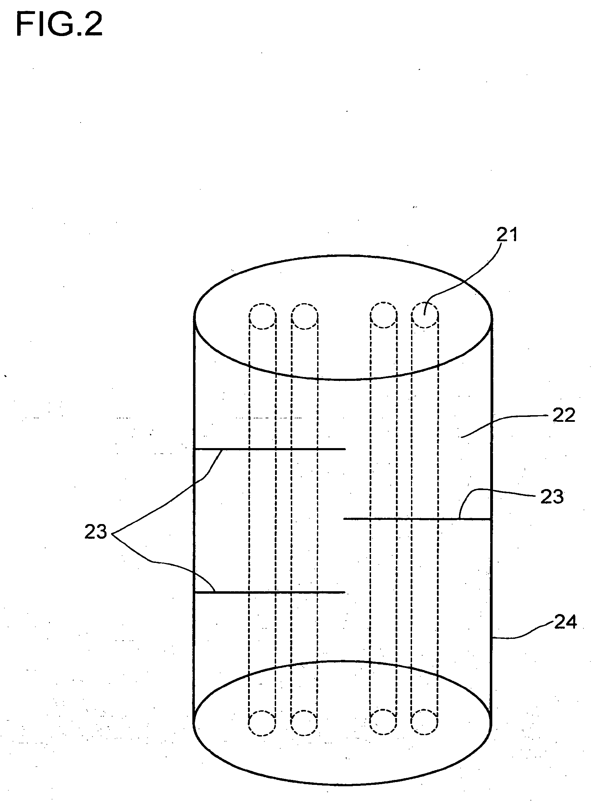

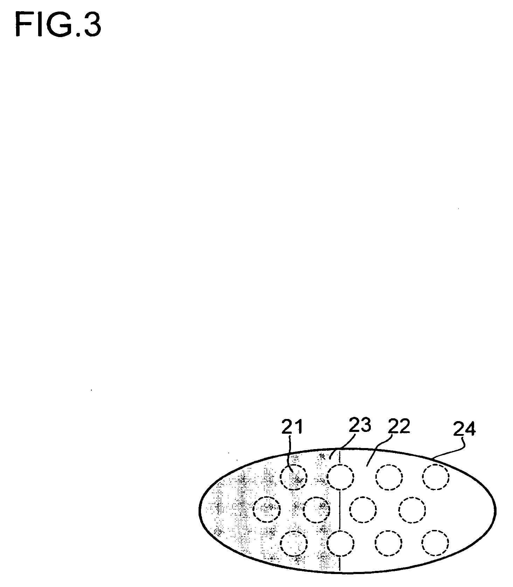

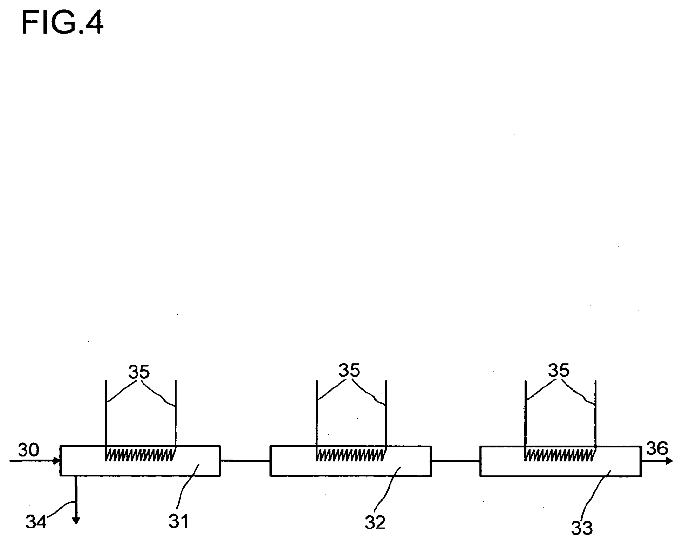

Im Folgenden wird die Erfindung anhand Zeichnungen näher beschrieben. Es zeigen
**Fig. 1** **-** Einen Verdampfer im Längsschnitt, worin die Bezugszeichen bedeuten:
   1: Ein -, bzw. Ausgang des Temperiermediums
   2: Anschlussflansch
   3: Dichtung
   4: Anschlussflansch
   5: Leitblech
   6: Einraumverdampfermantel
   7: Temperierkanal
   8: Sechskantschraube
   9: Sechskantmutter
**Fig. 2** **und** **Fig. 3** **-** Einen Verdampfer im Schnitt, worin die Bezugszeichen bedeuten:
   21: Heizkanäle
   22: Mantelraum
   23: Leitbleche
   24: Apparatemantel
**Fig. 4** **-** Fließschema zur Verdampfung und Reaktion, worin bedeuten:
   30: Verdampferzulauf
   31 Einraumverdampfer
   32: Vorheizer
   33: Dehydratisierungsreaktor
   34: Purgestrom
   35: Temperierseite des Apparates, Heizmedium z. B. Dampf, Wälzgas oder flüssige Wärmeträgermedien
   36: Reaktorausgang

In einer bevorzugten Ausführungsform betrifft die Erfindung ein Verfahren zur Herstellung von Blausäure (HCN) durch katalytische. Dehydratisierung von gasförmigem Formamid in Anwesenheit von Luftsauerstoff, wobei das Formamid in einem erfindungsgemäßen Einraumverdampfer zunächst bei Drücken oberhalb von 600 mbar und Temperaturen oberhalb von 200 °C mit Ausbeuteverlusten < 1 % verdampft wird: Verfahrensbeschreibung

### Schritt i)

Formamid wird mit Hilfe eines Einraumverdampfers verdampft. Aus dem Verdampfungsraum kann ein Purgestrom zur Entfernung von Polymerisatresten oder sonstigen in Spuren im Formamid enthaltenen Metallen bzw. höhersiedenden Bestandteilen entnommen werden. Der Purgestrom kann gemäß dem Stand der Technik gereinigt und dem Verdampfer wieder zugeführt werden.

### Schritt ii)

Der Formamiddampf wird anschließend in einem Reaktor gemäß dem Stand der Technik zu HCN umgesetzt. Dem dampfförmigen Formamid wird im Allgemeinen Luft zugeführt. Der Luftanteil kann gegebenenfalls im vorgewärmten Zustand zugeführt werden. Wesentlich für eine hohe Selektivität ist, dass der eigentliche Zersetzungs-Apparat eine Fe-haltige Oberfläche besitzt.

Im Allgemeinen wird dem Verdampfer flüssiges Formamid zugeführt. Dieses wird in Schritt i) des erfindungsgemäßen Verfahrens zu gasförmigem Formamid verdampft, welches anschließend in der katalytischen Dehydratisierung in Schritt ii) des erfindungsgemäßen Verfahrens eingesetzt wird.

Bevorzugt wird das Formamid in Schritt i) des erfindungsgemäßen Verfahrens vollständig (restlos) verdampft. Besonders bevorzugt wird das Formamid in Schritt i) vollständig verdampft und der entstehende Formamiddampf auf Temperaturen von im Allgemeinen 230°C oder mehr überhitzt. Der überhitzte Formamiddampf kann direkt in Schritt ii) eingesetzt werden.

Vor der Zuführung des in Schritt i) erhaltenen gasförmigen Formamids in Schritt ii) des erfindungsgemäßen Verfahrens kann dem gasförmigen Formamid Sauerstoff z. B. in Form von Luftsauerstoff oder in Form eines Sauerstoff enthaltenden Gasgemisches zugeführt werden, wobei der Sauerstoffanteil gegebenenfalls in einem vorgewärmten Zustand zugeführt werden kann.

In einer bevorzugten Ausführungsform wird Schritt ii) des erfindungsgemäßen Verfahrens in Anwesenheit von Sauerstoff, bevorzugt Luftsauerstoff, durchgeführt. Die Mengen an Sauerstoff, bevorzugt Luftsauerstoff, betragen im Allgemeinen > 0 bis 10 mol-%, bezogen auf die eingesetzte Formamidmenge, bevorzugt 0,1 bis 10 mol-%, besonders bevorzugt 0,5 bis 3 mol-%.

Anschließend kann das gasförmige Formamid (Formamid-Dampf) bzw. das Formamid-Sauerstoff-Gemisch, bevorzugt das Formamid-Luft-Gemisch, in einem Wärmetauscher auf Temperaturen von 350°C oder mehr gebracht werden, bevor es Schritt ii) zugeführt wird. Es ist jedoch ebenfalls möglich, den vorstehend erwähnten in Schritt i) erhaltenen gering überhitzten Formamiddampf direkt, ggf. nach Zumischung von Sauerstoff, in Schitt ii) einzusetzen.

Die katalytische Dehydratisierung in Schritt ii) des erfindungsgemäßen Verfahrens erfolgt im Allgemeinen bei Temperaturen von 350 bis 650°C, bevorzugt 380 bis 550°C, besonders bevorzugt 440 bis 510°C. Werden aber höhere Temperaturen gewählt, ist mit verschlechterten Selektivitäten zu rechnen.

Der Druck in Schritt ii) des erfindungsgemäßen Verfahrens beträgt im Allgemeinen 70 mbar bis 5 bar, bevorzugt 3 bar, insbesondere 2 bar, ganz besonders bevorzugt 400 mbar bis 2,0 bar und speziell bevorzugt 600 mbar bis 1,5 bar.

### Reaktor zur Dehydratisierung

Als Reaktor können in Schritt ii) des erfindungsgemäßen Verfahrens alle dem Fachmann zur Dehydratisierung von Formamid bekannten Reaktoren eingesetzt werden. Bevorzugt werden in Schritt ii) des erfindungsgemäßen Verfahrens Rohrreaktoren eingesetzt, wobei geeignete Rohrreaktoren dem Fachmann bekannt sind. Besonders bevorzugt handelt es sich bei den Rohrreaktoren um Mehrrohrreaktoren. Geeignete Mehrrohrreaktoren sind dem Fachmann ebenfalls bekannt.

Geeignete Materialien der in Schritt ii) des erfindungsgemäßen Verfahrens eingesetzten Reaktoren sind dem Fachmann ebenfalls bekannt. Bevorzugt wird als innere Oberfläche des Reaktors eine eisenhaltige Oberfläche eingesetzt. In einer besonders bevorzugten Ausführungsform ist die innere Reaktoroberfläche aus Stahl aufgebaut, der besonders bevorzugt Eisen sowie Chrom und Nickel enthält. Der Anteil an Eisen in dem bevorzugt die innere Reaktoroberfläche bildenden Stahl beträgt im Allgemeinen > 50 Gew.-%, bevorzugt > 60 Gew.-%, besonders bevorzugt > 70 Gew.-%. Der Rest sind im Allgemeinen Nickel und Chrom, wobei gegebenenfalls geringe Mengen weiterer Metalle wie Molybdän, Mangan, Silizium, Aluminium, Titan, Wolfram, Kobalt mit einem Anteil von im Allgemeinen 0 bis 5 Gew.-%, bevorzugt 0 bis 2 Gew.-%, enthalten sein können. Für die innere Reaktoroberfläche geeignete Stahlqualitäten sind im Allgemeinen Stahlqualitäten entsprechend den Normen 1.4541, 1.4571, 1.4573, 1.4580, 1.4401, 1.4404, 1.4435, 2.4816, 1.3401, 1.4876 und 1.4828. Bevorzugt werden Stahlqualitäten entsprechend den Normen 1.4541, 1.4571, 1.4828, 1.3401, 1.4876 und 1.4762, besonders bevorzugt Stahlqualitäten entsprechend den Normen 1.4541, 1.4571, 1.4762 und 1.4828 eingesetzt.
Mithilfe eines solchen Rohrreaktors ist eine katalytische Dehydratisierung von gasförmigem Formamid zu Blausäure in Schritt ii) des erfindungsgemäßen Verfahrens möglich, ohne dass zusätzliche Katalysatoren eingesetzt werden müssen bzw. der Reaktor zusätzliche Einbauten aufweist.

Es ist jedoch ebenfalls möglich, dass die katalytische Dehydratisierung in Schritt ii) des erfindungsgemäßen Verfahrens in Anwesenheit von Formkörpern als Katalysatoren durchgeführt wird, wobei die Formkörper bevorzugt hoch gesinterte Formkörper, aufgebaut aus Aluminiumoxid und gegebenenfalls Siliziumoxid sind, bevorzugt aus 50 bis 100 Gew.-% Aluminiumoxid und 0 bis 50 Gew.-% Siliziumoxid, besonders bevorzugt aus 85 bis 95 Gew.-% Aluminiumoxid und 5 bis 15 Gew.-% Siliziumoxid, oder aus Chrom-Nickel-Edelstahl, wie z. B. in EP-A 0 209 039 beschrieben. Des Weiteren kann es sich bei geeigneten in Schritt ii) des erfindungsgemäßen Verfahrens eingesetzten Katalysatoren um Packungen aus Stahl oder Eisenoxid auf porösen Trägermaterialien, z. B. Aluminiumoxid, handeln. Geeignete Packungen sind z. B. in DE-A 101 38 553 beschrieben.

Werden Formkörper eingesetzt, so können als mögliche Formkörper sowohl geordnete als auch ungeordnete Formlinge eingesetzt werden, z. B. Raschig-Ringe, Pal-Ringe, Tabletten, Kugeln und ähnliche Formlinge. Wesentlich ist hierbei, dass die Packungen bei mäßigem Druckverlust guten Wärmeübergang ermöglichen. Die Größe bzw. Geometrie der verwendeten Formlinge richtet sich im Allgemeinen nach dem Innendurchmesser der mit diesen Formkörpern zu füllenden Reaktoren, bevorzugt Rohrreaktoren.

Geeignete Packungen aus Stahl oder Eisenoxid sind im Allgemeinen geordnete Packungen. Bevorzugt handelt es sich bei den geordneten Packungen um statische Mischer. Durch den Einsatz der statischen Mischer kann ein einheitlicher Druck sowie ein hervorragender Wärmeübergang im Rohrreaktor erreicht werden. Die statischen Mischer können beliebige Geometrien aufweisen, wie sie dem Fachmann bekannt sind. Bevorzugte statische Mischer sind aus Blechen aufgebaut, wobei es sich um Lochbleche und/oder geformte Bleche handeln kann. Es können selbstverständlich ebenfalls geformte Lochbleche eingesetzt werden.

Geeignete Formkörper sind in EP-A 0 209 039 beschrieben und geeignete statische Mischer sind in DE-A 101 38 553 beschrieben.

Es ist ebenfalls möglich, dass in Schritt ii) des erfindungsgemäßen Verfahrens ein Reaktor, bevorzugt ein Rohrreaktor, eingesetzt wird, der Formkörper und/oder Packungen aus Stahl oder Eisenoxid auf einem porösen Träger aufweist, dessen Reaktorwand zusätzlich katalytisch aktiv ist. Geeignete Reaktorwand-Materialien, die in Schritt ii) des erfindungsgemäßen Verfahrens katalytisch aktiv sind, sind vorstehend genannt und z. B. in WO 02/070588 beschrieben.

Die optimale Verweilzeit des Formamid-Gasstroms in Schritt ii) des erfindungsgemäßen Verfahrens ergibt sich bei Einsatz eines Rohrreaktors aus der flächenspezifischen Formamidbelastung, die im Allgemeinen 0,1 bis 100 kg/m², bevorzugt 2 bis 50 kg/m², besonders bevorzugt 4 bis 30 kg/m² beträgt. Die Dehydratisierung erfolgt vorzugsweise im Bereich laminarer Strömung.

Das erfindungsgemäße Verfahren zur Herstellung von Blausäure liefert die gewünschte Blausäure in hohen Selektivitäten von im Allgemeinen > 85%, bevorzugt > 90% und Umsätzen von im Allgemeinen > 70%, bevorzugt > 80%, so dass Ausbeuten von im Allgemeinen > 60%, bevorzugt > 75%, besonders bevorzugt > 90% erreicht werden.

### Beispiel

Ein Einraumverdampfer gemäß folgender Spezifikation wurde verwendet:
Einraumverdampfer:
   Mantelinnendurchmesser: 29,7 mm
   Hydraulischer Durchmesser: 17,2 mm
   Länge des Verdampferraumes: 200 mm
   Anzahl der Heizkanäle: 37
   Länge insgesamt:
   Innen-/Außendurchmesser der Heizkanäle: 1,77 bzw. 3,17 mm
   Abstand der Heizkanäle bezogen auf deren Mittelpunkt: 4mm
   Äußere Fläche der Temperierkanäle/freies Volumen des Verdampfungsraumes: 919 m²/m³

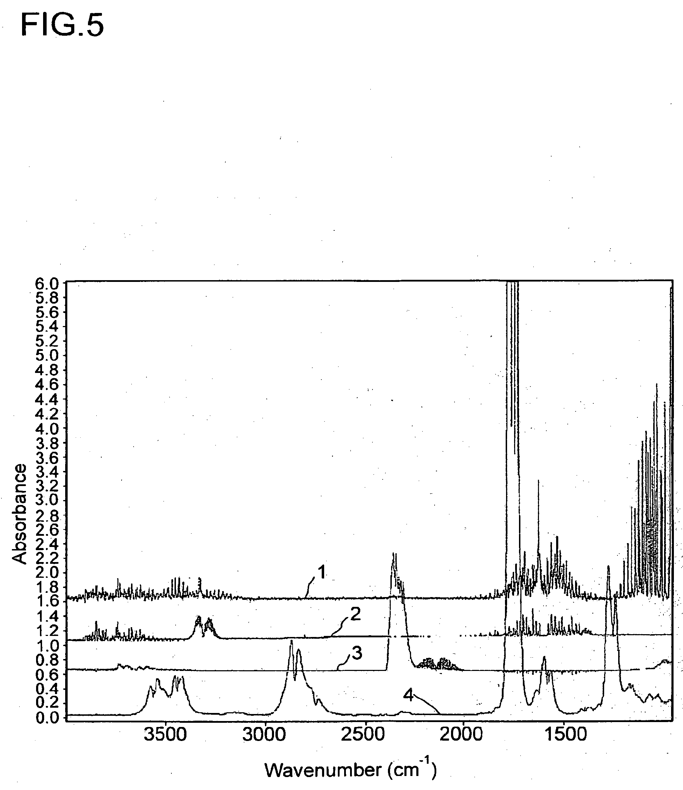

Formamid wurde im oben beschriebenen Verdampfer bei 800 mbar verdampft. Als Temperiermedium wurde Wasserdampf bei einem Druck von 45 bar verwendet. Der Dampf wurde überhitzt bei 270 °C den Temperierkanälen zugefahren. Die zugefahrene Formamidmenge betrug 7 kg/h. Die vollständige Verdampfung des Formamids wurde durch eine signifikante Überhitzung des Formamiddampfes mittels Temperaturmessung am Einraumverdampferausgang sichergestellt. Beim gefahrenen Druck von 800 mbar beträgt der Siedepunkt des Formamids ca. 210 °C. Die Austrittstemperatur des Formamids betrug 245 °C, die Überhitzung demnach 35°C. Unter diesen Bedingungen konnten keinerlei gasförmige Zersetzungsprodukte im IR-Spektrum detektiert werden, siehe Fig. 5. Figur 5 zeigt die IR-Spektren der reinen Komponenten, worin bedeuten:
1) NH₃
2) HCN
3) Gemisch CO₂ und CO
4) IR-Spektrum des Einraumverdampferabgases

## Patentansprüche

1. Verfahren zur Herstellung von Blausäure durch Verdampfen von Formamid in einem Verdampfer, Überführung des verdampften Formamids in einen Reaktor C, in welchem das Formamid in der Dampfphase unter katalytischer Dehydratisierung reagiert, **dadurch gekennzeichnet, dass** der Verdampfer im Wesentlichen einen Behälter und im Behälter Heizrohre HR aufweist, die vorzugsweise parallel zueinander und zur Längsachse des Verdampfers verlaufen, wobei der Behälter vorzugsweise aus nur einem Raum R besteht, wobei weiter
a) die auf den Raum R bezogene äußere Fläche der Heizrohre HR wenigstens 300 m²/m³ beträgt.
b) der hydraulische Durchmesser des Raumes R 0,01 bis 1 m beträgt,
c) der Raum eine Länge von 0,1 bis 5 m aufweist,

2. Verfahren nach Anspruch 1, **dadurch gekennzeichnet, dass** das Formamid bei wenigstens 600 mbar und einer Temperatur von wenigstens 200 °C verdampft wird.

3. Verfahren nach wenigstens einem der vorhergehenden Ansprüche, **dadurch gekennzeichnet, dass** der innere Durchmesser der Heizrohre HR 100 bis 6000 µm und der engste Abstand der Heizrohre HR maximal 3 mm beträgt.

4. Verfahren nach Anspruch 1, **dadurch gekennzeichnet, dass** die katalytische Dehydratisierung bei Temperaturen von 350 bis 650 °C erfolgt.

5. Verfahren nach Anspruch 1, **dadurch gekennzeichnet, dass** die katalytische Dehydratisierung bei einem Druck von 70 mbar bis 5 bar Absolutdruck durchgeführt wird.

6. Verfahren nach Anspruch 5, **dadurch gekennzeichnet, dass** die Dehydratisierung bei einem Druck von 600 mbar bis 1,5 bar Absolutdruck durchgeführt wird.

7. Verfahren nach wenigstens einem der Ansprüche 1 bis 6, **dadurch gekennzeichnet, dass** die katalytische Dehydratisierung in einem Rohrreaktor, bevorzugt einem Mehrrohrreaktor, erfolgt.

8. Verfahren nach wenigstens einem der Ansprüche 1 bis 7, **dadurch gekennzeichnet, dass** die katalytische Dehydratisierung in Anwesenheit von Formkörpern, ausgewählt aus hoch gesinterten Formkörpern, aufgebaut aus Aluminiumoxid und gegebenenfalls Siliziumoxid und Chrom-Nickel-Edelstahl-Formkörpern oder in Anwesenheit von Packungen aus Stahl oder Eisenoxid auf porösen Trägermaterialien als Katalysatoren erfolgt und/oder die innere Reaktoroberfläche des Rohrreaktors aus Stahl aufgebaut ist und als Katalysator dient.

9. Verfahren nach wenigstens einem der Ansprüche 1 bis 8, **dadurch gekennzeichnet, dass** die katalytische Dehydratisierung in Anwesenheit von Sauerstoff durchgeführt wird.

10. Verfahren nach einem der Ansprüche 7 bis 9, **dadurch gekennzeichnet, dass** die katalytische Dehydratisierung bei einer flächenspezifischen Formamid-Belastung von 0,1 bis 100 kg/m² im Bereich laminarer Strömung erfolgt.

11. Einraumverdampfer zur Verwendung in einem Verfahren gemäß wenigstens einem der Ansprüche 1 bis 10, **dadurch gekennzeichnet, dass** der Verdampfer im Wesentlichen einen Behälter und im Behälter Heizrohre HR aufweist, die vorzugsweise parallel zueinander und zur Längsachse des Verdampfers verlaufen, wobei der Behälter vorzugsweise aus nur einem Raum R besteht, wobei weiter
a) die auf den Raum R bezogene äußere Fläche der Heizrohre HR wenigstens 300 m²/m³ beträgt.
b) der hydraulische Durchmesser des Raumes R 0,01 bis 1 m beträgt
c) der Raum eine Länge von 0,1 bis 5 m aufweist und der innere Durchmesser der Heizrohre HR 100 bis 6000 µm beträgt.

12. Verwendung eines Einraumverdampfers gemäß Anspruch 11 zur Verdampfung von Formamid in einem Verfahren zur Herstellung von Blausäure aus Formamid.

## Claims

1. A process for preparing hydrogen cyanide by vaporizing formamide in a vaporizer, transferring the vaporized formamide to a reactor C in which the formamide reacts in the vapor phase under catalytic dehydration, wherein the vaporizer has essentially one vessel and heating tubes HR within the vessel, which preferably run parallel to one another and to the longitudinal axis of the vaporizer, where said vessel preferably consists only of one chamber R, and where
a) the outer area of the heating tubes HR based on the chamber R is at least 300 m²/m³,
b) the hydraulic diameter of the chamber R is 0.01 to 1 m,
c) the chamber has a length of 0.1 to 5 m.

2. The process according to claim 1, wherein the formamide is vaporized at at least 600 mbar and at a temperature of at least 200°C.

3. The process according to at least one of the preceding claims, wherein the internal diameter of the heating tubes HR is 100 to 6000 µm and the shortest distance between the heating tubes HR is not more than 3 mm.

4. The process according to claim 1, wherein the catalytic dehydration is effected at temperatures of 350 to 650°C.

5. The process according to claim 1, wherein the catalytic dehydration is performed at a pressure of 70 mbar to 5 bar absolute.

6. The process according to claim 5, wherein the dehydration is performed at a pressure of 600 mbar to 1.5 bar absolute.

7. The process according to at least one of claims 1 to 6, wherein the catalytic dehydration is effected in a tubular reactor, preferably a multitube reactor.

8. The process according to at least one of claims 1 to 7, wherein the catalytic dehydration is effected in the presence of shaped bodies selected from highly sintered shaped bodies formed from aluminum oxide and optionally silicon oxide and chromium-nickel stainless steel shaped bodies, or in the presence of packings composed of steel or iron oxide on porous support materials as catalysts, and/or the inner reactor surface of the tubular reactor is formed from steel and serves as a catalyst.

9. The process according to at least one of claims 1 to 8, wherein the catalytic dehydration is performed in the presence of oxygen.

10. The process according to any of claims 7 to 9, wherein the catalytic dehydration is effected at a superficial formamide velocity of 0.1 to 100 kg/m² in the laminar flow range.

11. A single-chamber vaporizer for use in a process according to at least one of claims 1 to 10, wherein the vaporizer has essentially one vessel and heating tubes HR within the vessel, which preferably run parallel to one another and to the longitudinal axis of the vaporizer, where said vessel preferably consists only of one chamber R, and where
a) the outer area of the heating tubes HR based on the chamber R is at least 300 m²/m³,
b) the hydraulic diameter of the chamber R is 0.01 to 1 m,
c) the chamber has a length of 0.1 to 5 m and the internal diameter of the heating tubes HR is 100 to 6000 µm.

12. The use of a single-chamber vaporizer according to claims 11 for vaporization of formamide in a process for preparing hydrogen cyanide from formamide.

## Revendications

1. Procédé de fabrication d'acide cyanhydrique par évaporation de formamide dans un évaporateur, transfert du formamide évaporé dans un réacteur C, dans lequel le formamide est mis en réaction dans la phase vapeur par déshydratation catalytique, **caractérisé en ce que** l'évaporateur comprend principalement un contenant et des tubes chauffants HR dans le contenant, qui sont de préférence parallèles les uns aux autres et à l'axe longitudinal de l'évaporateur, le contenant n'étant de préférence constitué que par une chambre R,
a) la surface extérieure par rapport à la chambre R des tubes chauffants HR étant d'au moins 300 m²/m³,
b) le diamètre hydraulique de la chambre R étant de 0,01 à 1 m,
c) la chambre présentant une longueur de 0,1 à 5 m.

2. Procédé selon la revendication 1, **caractérisé en ce que** le formamide est évaporé à au moins 600 mbar et à une température d'au moins 200 °C.

3. Procédé selon au moins l'une quelconque des revendications précédentes, **caractérisé en ce que** le diamètre intérieur des tubes chauffants HR est de 100 à 6 000 µm et l'écart le plus étroit des tubes chauffants HR est d'au plus 3 mm.

4. Procédé selon la revendication 1, **caractérisé en ce que** la déshydratation catalytique a lieu à des températures de 350 à 650 °C.

5. Procédé selon la revendication 1, **caractérisé en ce que** la déshydratation catalytique est réalisée à une pression de 70 mbar à 5 bar de pression absolue.

6. Procédé selon la revendication 5, **caractérisé en ce que** la déshydratation est réalisée à une pression de 600 mbar à 1,5 bar de pression absolue.

7. Procédé selon au moins l'une quelconque des revendications 1 à 6, **caractérisé en ce que** la déshydratation catalytique a lieu dans un réacteur tubulaire, de préférence un réacteur à plusieurs tubes.

8. Procédé selon au moins l'une quelconque des revendications 1 à 7, **caractérisé en ce que** la déshydratation catalytique a lieu en présence de corps moulés choisis parmi les corps moulés hautement frittés, formés par de l'oxyde d'aluminium et éventuellement de l'oxyde de silicium et les corps moulés en acier inoxydable au chrome-nickel, ou en présence de garnissages en acier ou en oxyde de fer sur des matériaux supports poreux en tant que catalyseurs, et/ou la surface intérieure du réacteur tubulaire est en acier et sert de catalyseur.

9. Procédé selon au moins l'une quelconque des revendications 1 à 8, **caractérisé en ce que** la déshydratation catalytique est réalisée en présence d'oxygène.

10. Procédé selon au moins l'une quelconque des revendications 7 à 9, **caractérisé en ce que** la déshydratation catalytique a lieu à un chargement en formamide spécifique à la surface de 0,1 à 100 kg/m² dans la plage d'un écoulement laminaire.

11. Évaporateur à une chambre, destiné à une utilisation dans un procédé selon au moins l'une quelconque des revendications 1 à 10, **caractérisé en ce que** l'évaporateur comprend principalement un contenant et des tubes chauffants HR dans le contenant, qui sont de préférence parallèles les uns aux autres et à l'axe longitudinal de l'évaporateur, le contenant n'étant de préférence constitué que par une chambre R,
a) la surface extérieure par rapport à la chambre R des tubes chauffants HR étant d'au moins 300 m²/m³,
b) le diamètre hydraulique de la chambre R étant de 0,01 à 1 m,
c) la chambre présentant une longueur de 0,1 à 5 m et le diamètre intérieur des tubes chauffants HR étant de 100 à 6 000 µm.

12. Utilisation d'un évaporateur à une chambre selon la revendication 11 pour l'évaporation de formamide dans un procédé de fabrication d'acide cyanhydrique à partir de formamide.
